# DEMANDE DE BREVET EUROPEEN

(11) **EP 4 632 763 A1**
(43) Date de publication de la demande: **15.10.2025**
(21) Numéro de dépôt: 25169660.5
(22) Date de dépôt: 10.04.2025
(51) Int. Cl.: G16H 50/20, A61B 5/18

(54) **PROCÉDÉ D'ÉVALUATION DU NIVEAU DE FATIGUE D'UN OPÉRATEUR ET SYSTÈME D'ÉVALUATION ASSOCIÉ**

(30) Priorité: 12.04.2024 FR 2403806
(71) Demandeur: THALES, 92190 Meudon (FR)
(72) Inventeur: GRANIER, Lionel, 33700 MERIGNAC (FR); DURIEUX, Florence, 33700 MERIGNAC (FR); DUMERCQ, Philippe, 33700 MERIGNAC (FR)
(74) Mandataire: Lavoix

(57) **Abrégé**

La présente invention concerne un procédé d'évaluation du niveau de fatigue d'un opérateur, le procédé comprenant les étapes suivantes mises en œuvre par un dispositif transportable d'évaluation (12) :
- identification (110) de l'opérateur ;
- acquisition ou détermination (120) de données contextuelles relatives au contexte dans lequel l'évaluation est mise en œuvre ;
- acquisition (130) de données physiologiques de l'opérateur ;
le procédé comprenant en outre les étapes suivantes :
- analyse (140) de l'ensemble des données acquises ou déterminées pour déterminer un niveau de fatigue de l'opérateur ;
- communication (150) du niveau de fatigue déterminé.

## Description

La présente invention concerne un procédé d'évaluation du niveau de fatigue d'un opérateur.

La présente invention concerne également un système d'évaluation du niveau de fatigue de l'opérateur qui met en œuvre ce procédé.

L'invention se situe dans le domaine technique de la collecte de données auprès d'un opérateur et de détermination de la fatigue de celui-ci.

L'opérateur évolue par exemple dans un contexte opérationnel critique. Autrement dit, la fatigue de l'opérateur dans ce contexte critique peut conduire à des conséquences importantes. Cela est le cas notamment dans le domaine aéronautique, aérospatial, nucléaire, de la médecine, etc.

Dans le domaine aéronautique, afin d'atténuer les risques induits par la fatigue notamment d'un pilote dans l'aviation commerciale, les compagnies aériennes mettent en places des procédures visant à mesurer et à contrôler la fatigue du personnel navigant (FRMS pour « Fatigue Risk Management System » en anglais).

Ces mesures portent notamment sur des évaluations subjectives de la fatigue perçue par les opérateurs, sous la forme d'auto-déclarations spontanées. Ces déclarations s'appuient sur des ressentis personnels pouvant être biaisés par des facteurs culturels, professionnels ou opérationnels.

L'état de la technique propose déjà quelques techniques permettant d'évaluer la fatigue d'un opérateur.

Ces solutions se basent principalement sur des modèles biomathématiques qui sont adaptés à des interactions en usage opérationnel. Elles visent notamment à la levée d'alerte en cas de détection d'un niveau de fatigue élevé et fournissent des données limitées sur le contexte des mesures.

En outre, ces solutions sont principalement destinées à un suivi individuel et ne permettent pas la centralisation et le croisement des données à l'échelle d'une population.

La présente invention vise à remédier à ces inconvénients en proposant une solution qui permet d'évaluer de manière objective le niveau de fatigue d'un opérateur, puis d'identifier des facteurs influençant l'évolution de son niveau de fatigue et plus largement, d'une population à laquelle il appartient.

À cet effet, l'invention a pour but un procédé d'évaluation du niveau de fatigue d'un opérateur, le procédé comprenant les étapes suivantes mises en œuvre par un dispositif transportable d'évaluation :
- identification de l'opérateur ;
- acquisition ou détermination de données contextuelles relatives au contexte dans lequel l'évaluation est mise en oeuvre ;
- acquisition de données physiologiques de l'opérateur ;
le procédé comprenant en outre les étapes suivantes :
- analyse de l'ensemble des données acquises ou déterminées pour déterminer un niveau de fatigue de l'opérateur ;
- communication du niveau de fatigue déterminé.

Le procédé selon l'invention permet ainsi non seulement d'évaluer de manière objective la fatigue d'un opérateur mais également d'identifier, de contextualiser, puis de centraliser les mesures effectuées à des fins de croisement à l'échelle d'une population.

L'invention permet ainsi de collecter et de traiter de manière centralisée les données relatives à la fatigue ainsi que les données contextuelles à l'échelle d'une population. Cela permet alors d'avoir une vue globale sur toute la population et ainsi de prendre la fatigue en compte de manière efficace pour planifier les activités et les missions des opérateurs.

Selon d'autres modes avantageux de l'invention, le procédé comprend l'une ou plusieurs des caractéristiques suivantes, prises isolément ou en combinaison selon toutes les combinaisons techniquement possibles :
- l'étape d'identification de l'opérateur comprend la mise en œuvre d'au moins l'une des techniques suivantes :
   - saisie d'un code personnel unique ;
   - lecture d'un support physique ou numérique externe ;
   - saisie d'un identifiant ou d'un mot de passe ;
   - reconnaissance d'une empreinte biométrique ;
- les données contextuelles comprennent au moins un type de données choisi dans le groupe comprenant :
   - données relatives à l'environnement de l'opérateur ;
   - données opérationnelles relatives à des activités effectuées par l'opérateur ;
   - données physiologiques de l'opérateur ;
- les données contextuelles sont saisies par l'opérateur et/ou acquises à partir d'un dispositif externe et/ou générées par le dispositif transportable d'évaluation ;
- l'étape d'acquisition de données physiologiques de l'opérateur est mise en œuvre à partir de mesures fournies par une pluralité de capteurs intégrés dans le dispositif transportable d'évaluation ;
- l'étape d'acquisition de données physiologiques comprend au moins l'une de sous-étapes suivantes :
   - vérification de l'état de fonctionnement des capteurs ;
   - synchronisation des capteurs ;
   - suivi de la qualité du signal mesuré ;
   - alerte en cas de défaillance d'un ou plusieurs capteurs ;
- l'étape d'acquisition de données physiologiques de l'opérateur est mise en œuvre lors d'une activité de l'opérateur autre que l'interaction avec le dispositif transportable d'évaluation, de préférence selon une durée minimale prédéterminée ;
- le niveau de fatigue de l'opérateur est déterminé par un ou plusieurs algorithmes de traitement des données physiologiques et avantageusement des données contextuelles ;
- l'étape de communication du niveau de fatigue comprend l'affichage de ce niveau de fatigue à l'opérateur et/ou le transfert de ce niveau de fatigue à un dispositif externe ;
- le procédé comprend en outre une étape de transfert sécurisé des données acquises et/ou déterminés à moins l'un des éléments choisi dans le groupe comprenant :
   - un dispositif de stockage externe ;
   - un dispositif mobile avec capacité de stockage ;
   - un serveur central ;
   - un autre dispositif transportable d'évaluation ;
- le procédé comprend en outre une étape d'analyse de fonctionnement du système transportable d'évaluation comprenant la vérification d'au moins un élément choisi dans le groupe comprenant :
   - espace de stockage disponible ;
   - performances de calcul ;
   - état d'une batterie interne ;
   - connectivité avec un serveur central et/ou avec un autre dispositif transportable d'évaluation ;
   - état de fonctionnement des composants matériels ou logiciels mis en œuvre lorsqu'un dysfonctionnement est détecté ;
- le procédé comprend en outre une étape de mise à jour au moins d'une partie du dispositif transportable d'évaluation choisie dans le groupe comprenant :
   - des logiciels mis en œuvre par le dispositif transportable d'évaluation ;
   - des algorithmes de traitement de données pour déterminer le niveau de fatigue ;
   - la nature des données contextuelles.

L'invention a également pour objet un système d'évaluation du niveau de fatigue d'un opérateur, comprenant des moyens configurés pour mettre en œuvre le procédé tel que défini ci-dessus.

Ces caractéristiques et avantages de l'invention apparaitront à la lecture de la description qui va suivre, donnée uniquement à titre d'exemple non limitatif, et faite en référence aux dessins annexés sur lesquels :
- [Fig. 1] la figure 1 est une vue schématique d'un système d'évaluation selon l'invention ;
- [Fig. 2] la figure 2 est une vue schématique d'un dispositif transportable d'évaluation faisant partie du système d'évaluation de la figure 1 ;
- [Fig. 3] la figure 3 est un organigramme d'un procédé d'évaluation selon l'invention, le procédé étant mis en œuvre par le système d'évaluation de la figure 1 ; et
- [Fig. 4] [Fig. 5] [Fig. 6] les figures 4 à 6 sont différentes illustrations de la mise en œuvre d'au moins certaines étapes du procédé de la figure 4.

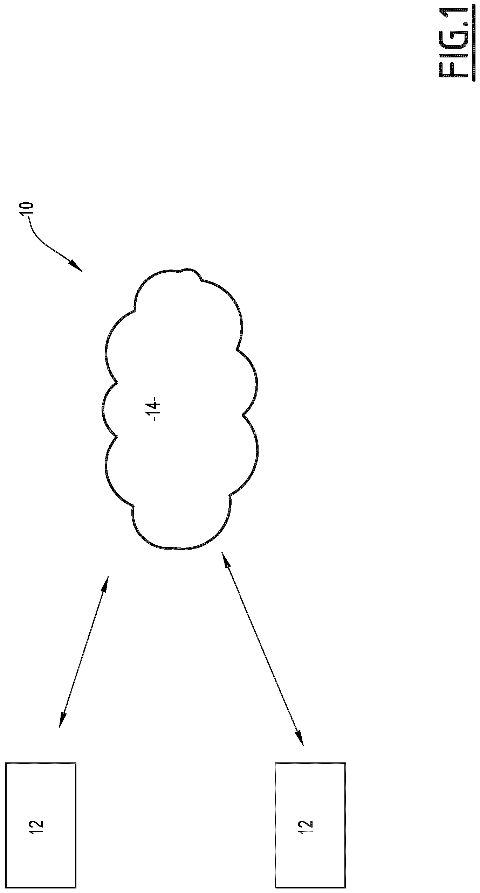

On a en effet illustré sur la figure 1 un système d'évaluation 10 du niveau de fatigue d'un opérateur.

Avantageusement, le système d'évaluation 10 est utilisable dans le domaine aéronautique. Dans un tel cas, l'opérateur fait partie du personnel navigant, notamment du personnel navigant commercial. Selon d'autres exemples, l'opérateur fait partie des opérateurs de planification de vols ou des opérateurs de maintenance ou des opérateurs de contrôle d'aéronef ou des contrôleurs aériens.

Avantageusement, l'opérateur est un pilote apte à piloter un aéronef.

Par aéronef, on entend tout engin volant pilotable à partir du cockpit de celui-ci, comme cela est le cas par exemple d'un avion ou d'un hélicoptère, ou alors à distance de celui-ci, comme cela est le cas par exemple d'un drone.

De manière générale, la notion de l'opérateur peut s'appliquer à toute autre personne effectuant une mission critique, par exemple dans le domaine de transport (ferroviaire ou poids lourd par exemple) ou dans le domaine nucléaire ou spatial, ou dans la médecine.

Comme indiqué précédemment, l'opérateur effectue une mission qui est déterminée par le domaine de son activité.

En particulier, la mission de l'opérateur comprend une pluralité de tâches définies selon les compétences de l'opérateur.

Lorsque l'opérateur est un pilote d'aéronef, sa mission consiste généralement à un pilotage de l'aéronef d'un point de départ à un point de sa destination.

Le système d'évaluation 10 selon l'invention permet de déterminer le niveau de fatigue de l'opérateur.

Préférentiellement, l'opérateur est affecté à une mission et le système d'évaluation détermine le niveau de fatigue de l'opérateur avant la mission de l'opérateur. Par conséquent, le procédé d'évaluation est ponctuel et sa mise en œuvre se fait avant que l'opérateur ne débute sa mission.

Préférentiellement, le système d'évaluation détermine le niveau de fatigue de l'opérateur avant et après la mission de l'opérateur. Par conséquent, le procédé d'évaluation est mis en œuvre ponctuellement une première fois avant que l'opérateur ne débute sa mission et deuxième fois après que l'opérateur ait terminé sa mission. Par exemple, les deux acquisition sont mises en œuvre au cours d'une phase de briefing avant et respectivement de debriefing après la mission.

En référence à la figure 1, le système d'évaluation 10 comprend au moins un dispositif transportable d'évaluation 12 et un serveur central 14 communiquant avec ce dispositif transportable d'évaluation 12.

Dans certains modes de réalisation, le système d'évaluation 10 comprend une pluralité de dispositifs transportables d'évaluation 12. Dans l'exemple de la figure 1, deux dispositifs transportables d'évaluation 12 sont illustrés.

Le serveur central 14 présente par exemple un ou plusieurs calculateurs qui sont adaptés pour communiquer avec le ou chaque dispositif transportable d'évaluation 12 de manière sécurisée.

La communication peut par exemple être mise en œuvre via une connexion directe entre ce serveur 14 et le ou chaque dispositif transportable d'évaluation 12 via par exemple l'une des interfaces filaires ou sans fil connues en soi (WIFI, Bluetooth, Ethernet, IR, etc.).

Alternativement, la communication peut se faire par l'intermédiaire d'un support de stockage amovible tel qu'une clé USB, disque dur externe ou tout autre dispositif mobile ayant une capacité de stockage, tel qu'une montre connectée.

Encore en variante, la communication peut se faire par l'intermédiaire d'une connexion internet ou de tout autre réseau global ou local.

Le serveur central 14 présente également une certaine capacité de calcul permettant notamment d'exécuter des applications qui sont par exemple stockées dans sa mémoire.

Enfin, le serveur central 14 dispose d'une capacité de stockage de données permettant de collecter les données de l'ensemble des dispositifs transportables d'évaluation 12 et de les garder pendant un temps de stockage prédéterminé.

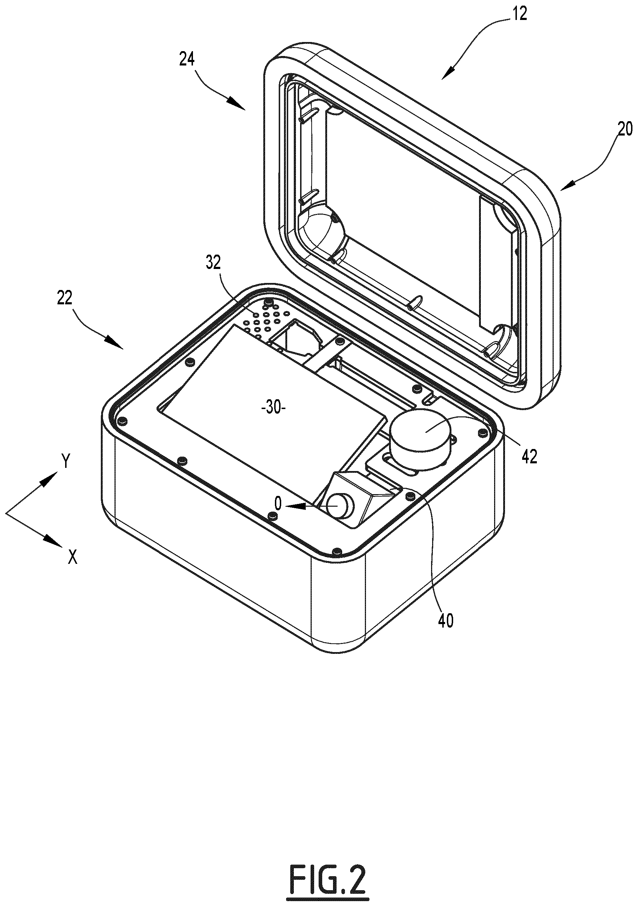

La figure 2 illustre plus en détail un exemple possible de réalisation d'un dispositif transportable d'évaluation 12.

Ainsi, et comme cela est représenté sur cette figure 2, le dispositif transportable d'évaluation 12 présente un boitier 20 intégrant différents composants internes de ce dispositif transportable d'évaluation 12.

En particulier, le boitier 20 se présente par exemple sous la forme d'une valise ou de tout autre objet pouvant être transporté facilement. Dans l'exemple de la figure 2, le boitier 12 est composé de deux demi-coques 22, 24. Le boitier 20 peut comprendre également tout autre dispositif facilitant sa transportation comme par exemple une poignée, des roues, etc.

Au moins l'une des demi-coques, par exemple la demi-coque 24, forme alors une ouverture du boitier 20. Cette demi-coque 24 est mobile entre une position fermée et une position ouverte. Dans la position ouverte, illustrée sur la figure 2, la demi-coque 24 permet alors d'accéder au moins partiellement aux composants internes du dispositif transportable d'évaluation 12.

De manière générale, le boitier 20 comprend une pluralité de composants accessibles par l'opérateur lorsque la demi-coque 24 est dans sa position ouverte et une pluralité de composants inaccessibles par l'opérateur dans toute position de la demi-coque 24.

Parmi les composants accessibles par l'opérateur, le dispositif transportable d'évaluation 12 comprend notamment des moyens d'interaction avec l'opérateur et une pluralité de capteurs.

Les moyens d'interaction avec l'opérateur comprennent notamment des moyens d'interaction visuels tels qu'un écran 30 et des moyens d'interaction auditifs tels que par exemple un haut-parleur 32. L'écran 30 et le haut-parleur 32 sont par exemple intégrés dans une surface intérieure de la demi-coque 22 qui est destinée à être protégée par la demi-coque 24 lorsque celle-ci est dans sa position fermée.

La pluralité de capteurs comprend tout capteur permettant d'acquérir des données physiologiques de l'opérateur.

En particulier, dans l'exemple de la figure 2, la pluralité de capteurs comprend une caméra 40 configurée pour acquérir des images de l'opérateur et un capteur 42 permettant de mesurer le rythme cardiaque de l'opérateur.

La caméra 40 est avantageusement orientée vers l'opérateur ou présente des moyens permettant de l'orienter selon la position de l'opérateur.

Le capteur 42 du rythme cardiaque de l'opérateur est avantageusement amovible du boitier 20 pour par exemple être positionné autour d'un poignet de l'opérateur.

À cet effet, le capteur 42 présente par exemple un bracelet susceptible d'être fixé sur le poignet de l'opérateur et une partie sensible qui est destinée à mesurer le rythme cardiaque de l'opérateur lorsque le bracelet est fixé sur son poignet.

La mesure du rythme cardiaque s'effectue par exemple par la partie sensible par la technique appelée photopléthysmographie, dit PPG. Alternativement, la partie sensible est configurée pour effectuer la mesure du rythme cardiaque à partir d'une analyse de réponse électrique par le poignet de l'opérateur ou par analyse de signaux de radar se propageant dans le poignet de l'opérateur.

Dans certains exemples, le capteur 42 est configuré pour mesurer d'autres paramètres physiologiques de l'opérateur tels que la pression artérielle, l'inspiration de l'oxygène, la sudation, le taux de déshydratation.

Pour la saturation en oxygène, le capteur 42 est par exemple configuré pour émettre en direction de la peau de l'opérateur et recevoir un signal lumineux comprenant au moins deux longueurs d'onde. Une première longueur d'onde correspondant à une longueur d'onde absorbée par les globules rouges saturés, une deuxième longueur d'onde correspondant à une longueur d'onde absorbée par les globules rouges non saturés. Pour déterminer la saturation en oxygène, le capteur 42 est alors configuré pour comparer l'intensité lumineuse reçue en réponse à chacune des deux longueurs d'onde.

De manière générale, le capteur 42 peut se présenter sous la forme d'une montre connectée qui peut alors être stockée dans le boitier 20 lorsque cela est nécessaire ou portée par l'opérateur pour par exemple mesurer son rythme cardiaque.

Bien entendu, les fonctionnalités précitées du capteur 42 peuvent former des capteurs séparés qui peuvent alors être disposés selon toutes configurations possibles à l'intérieur du boitier 20 ou alors sur une surface de celui-ci.

Les composants non accessibles du boitier 20 sont notamment disposés dans la partie intérieure du boitier 20, par exemple dans la partie intérieure de la demi-coque 22 et comprennent notamment un calculateur, une mémoire et un module d'alimentation.

Le calculateur comprend notamment un processeur permettant d'exécuter une pluralité d'applications qui sont stockées par exemple dans la mémoire du boitier.

Le calculateur comprend en outre des moyens de communication avec des dispositifs extérieurs, notamment avec d'autres dispositifs transportables d'évaluation 12 ou alors avec le serveur 14.

Le module d'alimentation permet d'alimenter l'ensemble des composants du dispositif transportable d'évaluation 12. Ce module comprend par exemple une batterie permettant d'alimenter de manière autonome ces composants. Cette batterie peut être associée à un dispositif de recharge permettant de brancher la batterie à un réseau électrique pour la recharger.

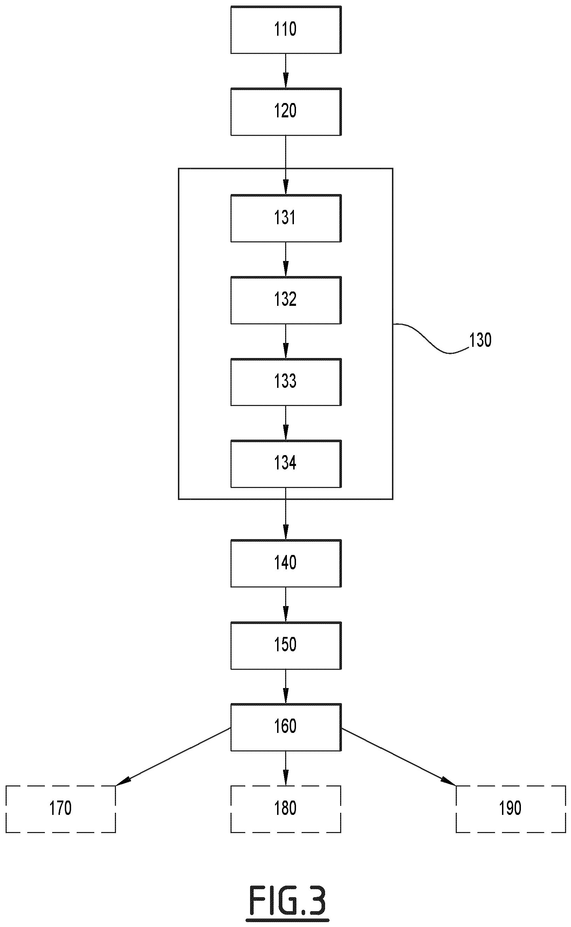

Le système d'évaluation 10 permet de mettre en œuvre un procédé d'évaluation selon l'invention qui sera désormais expliqué en référence à la figure 3 présentant un organigramme de ses étapes.

Il est considéré tout d'abord que le dispositif transportable d'évaluation 12 est disposé devant l'opérateur.

Lorsque ce dispositif transportable d'évaluation 12 se présente sous la forme d'une valise, la demi-coque 24 se trouve alors dans une position ouverte de sorte que l'opérateur puisse accéder à l'écran 30 et à la pluralité de capteurs de ce dispositif 12.

Lorsque le capteur 42 se présente sous la forme d'une montre, l'opérateur la met alors sur son poignet.

Il est considéré également que la caméra 40 est orientée vers l'opérateur.

Le fonctionnement du dispositif 12 est par exemple activé par un bouton ou toute autre commande prévue à cet effet à l'ouverture de la demi-coque 24.

Le procédé d'évaluation comprend une étape initiale 110 consistant à identifier l'opérateur par le dispositif transportable d'évaluation 12.

Pour ce faire, et préalablement à la mise en œuvre du procédé, l'opérateur se trouve attribué un code d'identification. Ce code d'identification permet d'associer les données collectées par le dispositif 12 lors des étapes suivantes à l'opérateur donné. De manière avantageuse, le code d'identification est associé à l'opérateur donné de manière anonymisée. Autrement dit, toute information collectée et associée à ce code d'identification ne permet pas d'identifier l'opérateur. Alternativement, les données collectées sont anonymisées uniquement avant l'envoi de ces données au serveur central 14.

Pour identifier l'opérateur, plusieurs solutions sont possibles.

Selon un premier exemple de réalisation, l'identification de l'opérateur comprend une saisie d'un code personnel unique qui est alors associé à l'opérateur.

Cette saisie peut se faire via une interface de saisie adaptée à cet effet ou via un dispositif de pointage couplé à un clavier numérique. Pour mettre en œuvre cette technique, il est nécessaire d'organiser au préalable l'attribution de codes d'identification uniques à l'ensemble des opérateurs susceptibles d'utiliser le dispositif transportable d'évaluation 12, sans conserver de trace des attributions pour l'anonymat de ces opérateurs.

Selon un deuxième exemple, l'identification de l'opérateur s'effectue via la lecture d'un support physique ou numérique externe. Ce support externe permet par exemple de garder un code personnel unique, éventuellement de manière cryptée. Les supports envisageables sont notamment :
- QR code si le dispositif transportable d'évaluation 12 est équipé d'une caméra de résolution suffisante ;
- support NFC si le dispositif 12 dispose d'un capteur de lecture adapté ;
- carte à puce si le dispositif 12 dispose d'un moyen de lecture des cartes à puce adapté ;
- tout autre dispositif amovible de stockage tel qu'une clé USB, CD, etc. que le dispositif 12 serait en mesure de lire.

Pour mettre en œuvre cette solution, il est nécessaire d'organiser au préalable des distributions de supports physiques ou numériques à l'ensemble des opérateurs susceptibles d'utiliser le dispositif 12 sans conserver de trace des attributions pour anonymat des opérateurs.

Selon un troisième exemple de réalisation, l'identification se fait par une saisie d'un identifiant et d'un mot de passe adaptés par l'opérateur. Cela est possible lorsque le dispositif 12 dispose d'une interface de saisie ou d'un dispositif de pointage couplé à un clavier numérique et d'un écran.

Pour mettre en œuvre cette solution, il convient à chaque opérateur de s'enregistrer, puis de conserver le mot de passe pour ses futures connexions.

Dans certains modes de réalisation, les identifiants et les mots de passe associés peuvent être configurés au préalable par le dispositif 12 lui-même. Ces éléments peuvent également être stockés sur le serveur 14 et redistribués au dispositif 12 à son démarrage.

Selon un quatrième exemple de réalisation, l'identification de l'opérateur se fait via une reconnaissance d'une empreinte biométrique.

Elle peut s'agir ici d'une méthode d'analyse d'empreinte digitale, de scan rétinien, de reconnaissance faciale ou vocale ou alors d'extraction de signature distinctive à partir de données physiologiques acquises. La signature distinctive peut être formée par une signature du rythme cardiaque, gestuel, saisie clavier, etc. Dans un tel cas, le dispositif transportable d'évaluation 12 est équipé d'un ou plusieurs capteurs permettant de reconnaitre une telle empreinte biométrique.

Bien entendu, les exemples précités permettant l'identification de l'opérateur peuvent être combinés entre eux au moins partiellement pour former une technique d'identification sécurisée empêchant toute usurpation d'identité.

Les techniques d'identification précitées peuvent également être renforcées par une procédure d'authentification multi-facteurs.

Cette procédure d'authentification multi-facteurs consiste par exemple à envoyer un code de confirmation sur un dispositif personnel de l'opérateur ayant l'accès à Internet. Cette solution est notamment réalisable lorsque le dispositif 12 est lui-même connecté également à Internet ou à tout autre réseau similaire.

Le procédé comprend en outre une étape 120 lors de laquelle le dispositif transportable d'évaluation 12 acquiert ou détermine des données contextuelles.

Les données contextuelles sont relatives au contexte dans lequel l'évaluation est mise en œuvre.

Ces données contextuelles peuvent comprendre par exemple au moins un type de donnée choisi dans le groupe comprenant :
- données relatives à l'environnement de l'opérateur ;
- données opérationnelles relatives à des activités effectuées par l'opérateur ;
- données physiologiques de l'opérateur.

Les données relatives à l'environnement de l'opérateur peuvent caractériser l'environnement dans lequel l'évaluation se fait. Ces données peuvent par exemple comprendre un horodatage (c'est-à-dire l'heure et la date) de l'évaluation, la géolocalisation de l'endroit où l'évaluation est faite, ainsi que la température et/ou l'humidité et/ou luminosité et/ou le bruit de l'endroit dans lequel l'évaluation est faite.

Les données opérationnelles relatives à des activités effectuées par l'opérateur peuvent par exemple comprendre une caractérisation de ces activités (par exemple la nature des activités, leur durée, etc.), difficultés ressenties par l'opérateur lors de ces activités, l'organisation de temps de repos de l'opérateur (par exemple la durée du sommeil, le nombre et la durée des siestes, la qualité de sommeil, etc.).

La collecte de données contextuelles peut être faite de différentes manières, en fonction notamment de la nature de celle-ci.

Par exemple, au moins certaines de ces données peuvent être saisies directement par l'opérateur, par exemple au moyen d'un dispositif de pointage ou de saisie via le remplissage dans le formulaire affiché sur l'écran 30, ou au moyen d'une page web ou application mobile associée au dispositif 12, ou par réponses orales à des questions posées par le dispositif 12. Dans ce dernier cas, une reconnaissance vocale des réponses prononcées par l'opérateur peut être utilisée.

Au moins certaines données contextuelles peuvent également être acquises par les capteurs du dispositif transportable d'évaluation 12 ou alors collectées depuis un autre système ou dispositif.

Ainsi, par exemple, il est possible de coupler une montre connectée ou de partager l'accès à un agenda numérique, une application de sport ou une application de centralisation de données de santé, au dispositif 12 pour collecter par exemple des données de suivi d'activités physiques, de suivi de sommeil ou d'aide à l'organisation de l'opérateur.

Il est également possible de coupler le dispositif 12 à l'organisme en charge de l'organisation des activités de l'utilisateur pour par exemple collecter des données relatives à la planification des interventions, des plannings de vols, etc.

Enfin, il est également possible d'utiliser une source d'information tierce telle qu'une application API qui présente une interface logicielle de suivi de vols commerciaux ou de rapports météorologiques.

Le procédé comprend en outre une étape 130 comprenant l'acquisition de données physiologiques de l'opérateur par le dispositif transportable d'évaluation 12.

Cette acquisition se fait en utilisant les capteurs intégrés dans le dispositif transportable d'évaluation 12.

Dans certains modes de réalisation, cette étape 130 comprend plusieurs sous-étapes permettant d'assurer le bon fonctionnement des capteurs.

Ainsi, par exemple, lors d'une première sous-étape 131 qui est mise en œuvre avant la collecte de données physiologiques, le dispositif 12 met en œuvre une vérification des capteurs.

Ainsi, par exemple, lorsqu'il s'agit d'un capteur amovible par rapport au dispositif 12, il convient de vérifier son appairage avec ce dispositif 12 ainsi que l'état de sa batterie. Il convient également de vérifier la compatibilité de la version du logiciel et de la configuration du capteur. Une mise à jour du capteur peut alors être appliquée. Un capteur obsolète peut être inhibé au cours de la mesure. Enfin, lors de cette sous-étape 131, l'état initial du capteur peut être rétabli afin de ne pas polluer l'acquisition des données actuelles avec celles d'un précédent utilisateur du dispositif 12.

Lors d'une sous-étape 132 suivante, le dispositif 12 synchronise l'ensemble des capteurs avec son horloge interne afin que toutes les données acquises soient horodatées avec la même référence. Le dispositif 12 peut également vérifier les conditions de fonctionnement de chaque capteur et notamment :
- le fait que les capteurs portés soient correctement installés par l'utilisateur ;
- le fait que l'utilisateur adopte la bonne posture et se positionne de la bonne distance des capteurs non-portés tels que la caméra 40 ;
- le fait que les capteurs ne soient pas affectés par des sources de pollution telles que lumières, bruits, vibrations, etc. pouvant altérer le fonctionnement de ceux-ci.

Les interfaces du dispositif 12 telles que l'écran 30 et/ou le haut-parleur 32 peuvent alors être utilisées pour accompagner l'utilisateur dans l'installation de ces capteurs et pour l'informer de l'avancement, de la réussite ou de l'échec de l'installation. Ensuite, l'acquisition des données physiologiques peut alors être effectuée.

Lors d'une sous-étape 133 mise en œuvre pendant par exemple l'acquisition des données physiologiques, le dispositif 12 met en œuvre un suivi de la qualité du signal mesuré de chaque capteur.

Plus particulièrement, dans cette sous-étape, la qualité des signaux et l'intégrité des capteurs sont régulièrement vérifiées afin d'informer l'utilisateur en cas de dégradation des conditions d'acquisition comme par exemple la position des capteurs portés, la position et la posture de l'utilisateur, la position des signaux, le disfonctionnement d'un capteur, l'autonomie restante, etc.

Si un capteur est impacté par des conditions externes, il peut faire l'objet d'un avertissement sonore ou visuel de l'opérateur.

Les interfaces du dispositif 12 permettent par ailleurs d'informer l'opérateur de la progression de l'acquisition des données physiologiques et de le notifier lorsque les données sont collectées. Ces interfaces peuvent également accompagner l'utilisateur dans la désinstallation des capteurs et leur mise en consigne. Par exemple, il convient de vérifier à ce stade que les capteurs fonctionnant sur batteries sont alimentés et d'inciter l'utilisateur à les remplacer sur leurs éventuels socles, stations d'accueil ou étuis de rangement.

Lorsqu'un ou plusieurs capteurs sont défaillants, l'étape d'acquisition 130 comprend une sous-étape 134 lors de laquelle une alerte est émise à destination de l'opérateur.

Avantageusement, l'étape d'acquisition de données physiologiques 130 est mise en œuvre par le dispositif 12 lorsque l'opérateur fait une activité qui est différente de l'interaction avec le dispositif transportable d'évaluation 12.

Par exemple, cette étape peut être mise en œuvre lorsque l'opérateur fait un débriefing de son activité future avec des collègues ou d'autres opérateurs, ou alors fait une activité habituelle relative à ses tâches habituelles.

Autrement dit, le but de cette étape est de mesurer les données physiologiques de l'opérateur dans son activité normale.

De préférence également, cette étape d'acquisition des données physiologiques est mise en œuvre selon une durée minimale prédéterminée. Cette durée minimale est par exemple égale à quelques minutes, par exemple à 5 minutes.

Avantageusement, la fin de cette étape d'acquisition des données physiologiques peut être également signalée par le dispositif 12 via les interfaces adaptées.

Par exemple, lorsque le dispositif 12 estime que les données physiologiques collectées sont suffisantes pour évaluer le niveau de fatigue de l'opérateur, un avertissement correspondant peut alors être émis.

Dans l'exemple décrit, les étapes suivantes sont mises en œuvre par le dispositif transportable d'évaluation 12. Toutefois selon un autre mode de réalisation, ces étapes peuvent également être mises en œuvre par le serveur central 14 ou alors par un autre dispositif transportable d'évaluation 12. Dans un tel cas, le procédé comprend en outre une étape de transmission des données contextuelles acquises ou déterminées lors de l'étape 120 ainsi que des données physiologiques acquises lors de l'étape 130 au serveur 14 ou alors à cet autre dispositif transportable d'évaluation 12.

Cette transmission s'effectue par exemple de manière sécurisée.

Également, avantageusement selon l'invention, avant d'être envoyées, ces données sont anonymisées si cela n'a pas été fait précédemment.

Autrement dit, avant d'envoyer ces données, le dispositif transportable d'évaluation 12 peut enlever de ces données toute trace permettant d'identifier l'opérateur.

Avantageusement, ces données sont transmises avec un identifiant anonymisé qui permet de déterminer que ces données appartiennent à une même personne pour éventuellement compléter ces données par la suite.

Lors de l'étape suivante 140, le dispositif transportable d'évaluation 12 analyse l'ensemble des données acquises ou déterminées lors des étapes précédentes, pour déterminer un niveau de fatigue de l'opérateur.

Pour cela, le dispositif 12 met en œuvre plusieurs algorithmes de traitement des données physiologiques et des données contextuelles. Selon certains modes de réalisation, les algorithmes choisis pour évaluer le niveau de fatigue de l'opérateur dépendent de la nature des données acquises ou déterminées lors des étapes précédentes.

Lorsque cette étape est mise en œuvre par le dispositif transportable d'évaluation 12, la détermination du niveau de fatigue s'effectue par exemple dans un temps réel.

En outre, le niveau de fatigue est avantageusement associé à une valeur prise sur une échelle de valeurs prédéterminée.

Cette échelle prédéterminée peut par exemple varier de 1 à 100 et le niveau de fatigue déterminé prend alors l'une des valeurs de l'intervalle allant de 1 à 100.

Lors de l'étape suivante 150, le dispositif transportable d'évaluation 12 communique le niveau de fatigue déterminé par exemple à l'opérateur ou à toute autre personne intéressée, telle que par exemple un supérieur de l'opérateur.

Cette communication comprend par exemple l'affichage du niveau de fatigue déterminé sur l'écran 30 du dispositif transportable d'évaluation 12 et/ou alors le transfert de ce niveau à un dispositif externe.

Lorsque ce niveau de fatigue est affiché sur l'écran 30, il peut faire par exemple l'objet d'un affichage particulier en fonction du niveau déterminé. Par exemple, différentes classes peuvent être identifiées sur l'échelle des niveaux de fatigue possibles. Lorsque le niveau déterminé tombe dans l'une de ces classes, l'affichage prend alors une forme qui est associée à cette classe.

Chaque classe peut par exemple correspondre à un niveau de fatigue modéré, élevé ou très élevé.

Par ailleurs, lorsque le niveau de fatigue déterminé tombe dans une classe particulière, par exemple dans la classe correspondant au niveau de fatigue très élevé, une alerte peut être levée.

Dans un tel cas, des recommandations opérationnelles ou des contre-mesures à appliquer peuvent être affichées à l'opérateur pour diminuer ce niveau ou alors pour éviter certaines tâches à venir.

Lors de l'étape 160 suivante, qui est mise en œuvre lorsque le dispositif transportable d'évaluation 12 a déterminé lui-même le niveau de fatigue de l'opérateur, il transfère les données acquises et/ou déterminées (y compris le niveau de fatigue) à au moins l'un des éléments choisis dans le groupe comprenant :
- un dispositif de stockage externe ;
- un dispositif mobile avec capacité de stockage tel qu'un téléphone portable ou une montre connectée ;
- le serveur distant 14 ;
- un autre dispositif transportable d'évaluation 12.

Le transfert effectué par le dispositif 12 est supervisé et l'opérateur peut être informé de sa progression, de son échec et de sa réussite.

Si une donnée est correctement transférée, elle est avantageusement supprimée du dispositif transportable d'évaluation 12. En cas d'échec de transfert, la donnée est conservée pour une tentative de transfert ultérieure. En cas d'échecs consécutifs, un mode de transfert alternatif peut être appliqué.

De préférence, le transfert est assuré en temps réel, immédiatement à la fin de la collecte et de détermination des données correspondantes. Le transfert peut faire l'objet d'un déclenchement automatique ou manuel.

Lorsque le transfert est effectué vers le serveur central 14, il convient de sécuriser ce transfert de manière particulière.

Par exemple, une première contre-mesure applicable pour sécuriser ce transfert est le filtrage par adresse IP qui n'autorise le transfert que depuis des connexions autorisées au préalable. Cette solution est simple à mettre en œuvre.

Une seconde contre-mesure consiste à protéger le transfert par authentification. Si le transfert s'effectue en temps réel ou immédiatement après la collecte et la détermination des données correspondantes, l'authentification s'appuie sur le mot de passe fourni par l'utilisateur. Si le transfert est déclenché automatiquement lors d'une période d'inactivité du dispositif 12, l'authentification s'appuie sur le mot de passe stocké sur celui-ci.

Le procédé peut comprendre en outre une étape optionnelle 170 d'analyse de fonctionnement du système transportable d'évaluation 12. Cette analyse est par exemple mise en œuvre par le dispositif 12 lui-même ou alors par le serveur central 14 ou un autre dispositif transportable d'évaluation 12.

Cette étape 170 est par exemple mise en œuvre lorsque le dispositif 12 n'est pas utilisé par l'opérateur, par exemple après que le niveau de fatigue de cet opérateur a été déterminé. Cette étape permet d'identifier les cas de panne ou d'anticiper les besoins en maintenance du dispositif 12.

Plus particulièrement, lors de cette étape, la vérification d'au moins un élément choisi dans le groupe suivant peut être effectuée :
- espace de stockage disponible ;
- performance de calcul (durée, précision, pertinence du résultat) ;
- intégrité des capteurs (cas de panne, perte en autonomie de la batterie) ;
- durée des transferts des données ;
- état d'une batterie interne ;
- connectivité avec le serveur central 14 ou un autre dispositif transportable d'évaluation 12.

Suite à la mise en œuvre de cette étape d'analyse, les actions de maintenance peuvent être réalisées si nécessaire. Il peut s'agir par exemple d'une intervention spécifique sur un système ou d'une action de maintenance générique planifiée (par exemple, suppression des données les plus anciennes stockées sur le dispositif si l'espace de stockage est restreint).

Dans certains modes de réalisation, le procédé comprend en outre une étape 180 de mise à jour d'au moins une partie du dispositif transportable d'évaluation 12. Cette partie est avantageusement choisie dans le groupe comprenant :
- des logiciels mis en œuvre par le dispositif 12 ;
- des algorithmes de traitement des données pour déterminer le niveau de fatigue ;
- la nature des données contextuelles.

Par exemple, la mise à jour peut adapter la nature des données contextuelles acquises ou déterminées lors de l'étape 120.

En outre, dans le cas d'un déploiement d'un grand nombre de dispositifs transportables d'évaluation 12 sur différents sites opérationnels, il convient de mettre en place une solution de distribution automatisée de mises à jour, avec un impact minimal sur la disponibilité de ces dispositifs. En outre, les dispositifs pouvant couvrir différents cas d'utilisation, il est nécessaire de maîtriser la version logicielle appliquée et le créneau de déploiement de ces mises à jour.

Dans un tel cas, par exemple une mise à jour de type OTA (de l'anglais « over the air ») peut se faire selon les sous-étapes suivantes :
- déploiement des composants logiciels sur un serveur de distribution (par exemple le serveur central 14) ;
- définition d'un sous-ensemble de composants logiciels versionnés définissant une livraison applicative cohérente ;
- test dans un environnement de validation de la livraison applicative ;
- affectation de la livraison applicative au sous-ensemble de dispositifs 12 ciblés par le déploiement de cette livraison ;
- le dispositif 12 vérifie régulièrement et spontanément la disponibilité d'une nouvelle livraison applicative à installer auprès du serveur de distribution ;
- le dispositif 12 télécharge la liste exhaustive des composants logiciels et leurs versions respectives à installer auprès du serveur de distribution ;
- le dispositif 12 archive la version courante de chaque composant à mettre à jour, télécharge puis installe la nouvelle version ;
- le dispositif 12 notifie le serveur de distribution de la progression de l'installation ;
- en cas d'échec d'installation d'un des composants, la mise à jour est annulée et un retour à la dernière version applicative stable est opéré.

En complément, une interface de pilotage des déploiements peut être couplée au serveur de distribution. Il est ainsi possible de s'assurer de la version couramment installée sur chaque système déployé sur un site opérationnel.

Dans certains modes de réalisation, le procédé peut également comprendre une étape 190 d'analyse de l'utilisation du dispositif 12. Cette étape est mise en œuvre pour analyser le comportement de l'opérateur face au dispositif, et cela dans le but de rendre l'utilisation du dispositif plus fluide et plus rapide et également pour améliorer la pertinence des données collectées.

En particulier, en observant les modes d'utilisation des interfaces et le temps nécessaire à chaque étape de la collecte de données, il est possible d'identifier celles qui sont les plus fastidieuses pour l'opérateur. Les étapes ainsi identifiées sont ensuite améliorées par le biais d'une mise à jour.

Cette analyse peut par exemple être faite en trois sous-étapes :
- chaque action sur une interface du dispositif 12 génère une trace ;
- les traces sont transférées vers le serveur centrale 14 ;
- les données centralisées sont croisées afin d'identifier les difficultés d'utilisation communes.

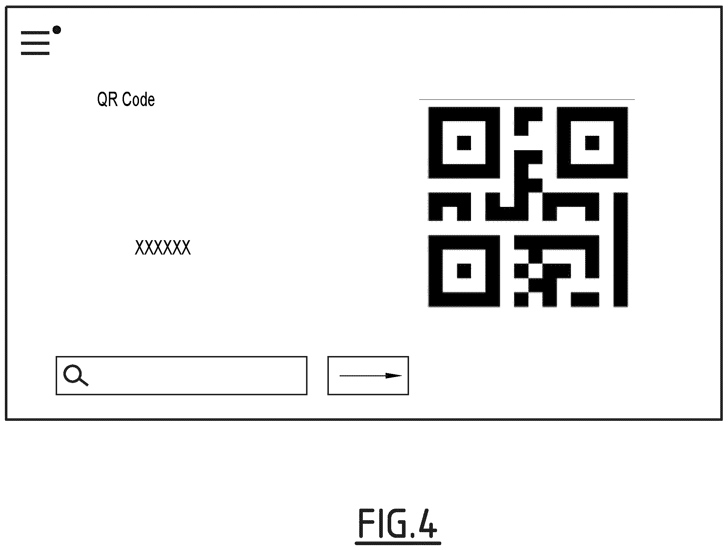

Les figures 4 à 6 illustrent le résultat de la mise en œuvre d'au moins certaines étapes du procédé expliqué précédemment.

Ainsi, par exemple, la figure 4 illustre un exemple de la mise en œuvre de l'étape 110 de l'identification de l'opérateur.

Selon cet exemple, l'opérateur présente par exemple un QR code sur un support physique devant la caméra 40 du dispositif 12. Ce QR code est alors reconnu et permet d'identifier l'opérateur. Dans un tel cas, l'affichage sur l'écran 30 peut être similaire à la figure 4.

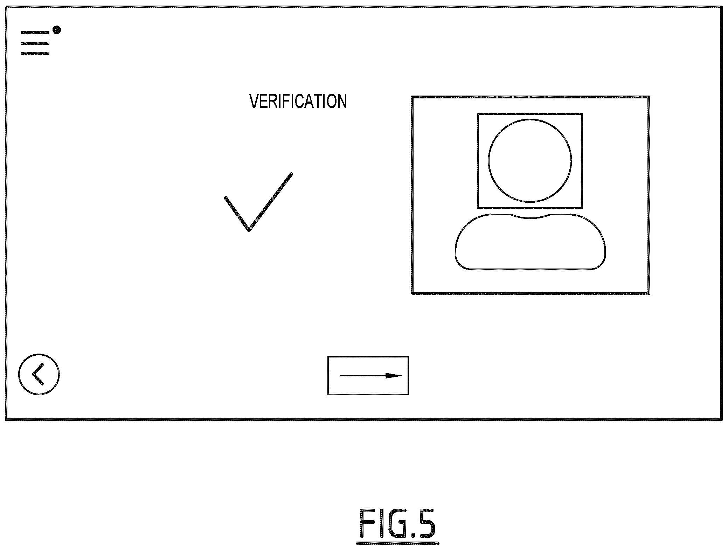

La figure 5 illustre un exemple de la mise en œuvre de l'étape 130 de l'acquisition des données physiologiques de l'opérateur.

Selon cet exemple, le dispositif 12 vérifie le positionnement de l'opérateur face à la caméra 40.

Lorsque ce positionnement est correct, l'écran 30 peut afficher une image analogue à celle de la figure 5.

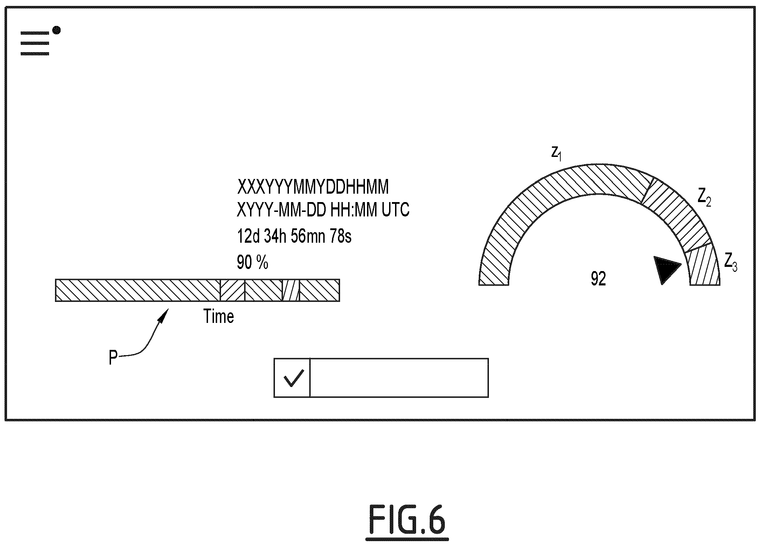

Enfin, la figure 6 illustre un exemple de la mise en œuvre de l'étape 150 de la communication du niveau de fatigue déterminé à l'opérateur.

Selon cette figure, trois classes de fatigue sont déterminées :
- modérée, correspondant à la zone Z1 de la figure 6 ;
- élevée, correspondant à la zone Z2 de la figure 6 ;
- très élevée, correspondant à la zone Z3 de cette figure 6.

Le niveau de fatigue déterminé peut être également affiché (92 dans l'exemple de la figure 6) avec l'indication de la classe à laquelle il appartient.

On conçoit alors que la présente invention présente un certain nombre d'avantages.

En particulier, l'invention permet la collecte de données nécessaires et suffisantes à la mesure d'un état physiologique et l'identification d'éventuels facteurs externes favorisant ou limitant cet état.

Le procédé selon l'invention est évolutif, ajustable à différents systèmes et chaque étape présentée peut être implémentée par différentes techniques.

Il doit être également compris que le niveau de fatigue déterminé par ce procédé doit être compris de manière large et inclut également d'autres états physiologiques tels que le niveau de stress, le niveau de la charge mentale, une mesure de performance physique, etc.

Bien entendu, d'autres modes de réalisation de l'invention sont également possibles.

## Revendications

1. Procédé d'évaluation du niveau de fatigue d'un opérateur, le procédé comprenant les étapes suivantes mises en œuvre par un dispositif transportable d'évaluation (12):
- identification (110) de l'opérateur ;
- acquisition ou détermination (120) de données contextuelles relatives au contexte dans lequel l'évaluation est mise en œuvre ;
- acquisition (130) de données physiologiques de l'opérateur ;
le procédé comprenant en outre les étapes suivantes :
- analyse (140) de l'ensemble des données acquises ou déterminées pour déterminer un niveau de fatigue de l'opérateur ;
- communication (150) du niveau de fatigue déterminé.

2. Procédé selon la revendication 1, dans lequel l'étape d'identification (110) de l'opérateur comprend la mise en œuvre d'au moins l'une des techniques suivantes :
- saisie d'un code personnel unique ;
- lecture d'un support physique ou numérique externe ;
- saisie d'un identifiant ou d'un mot de passe ;
- reconnaissance d'une empreinte biométrique.

3. Procédé selon la revendication 1 ou 2, dans lequel les données contextuelles comprennent au moins un type de données choisi dans le groupe comprenant :
- données relatives à l'environnement de l'opérateur ;
- données opérationnelles relatives à des activités effectuées par l'opérateur ;
- données physiologiques de l'opérateur.

4. Procédé selon l'une quelconque des revendications précédentes, dans lequel les données contextuelles sont saisies par l'opérateur et/ou acquises à partir d'un dispositif externe et/ou générées par le dispositif transportable d'évaluation (12).

5. Procédé selon l'une quelconque des revendications précédentes, dans lequel l'étape d'acquisition (130) de données physiologiques de l'opérateur est mise en œuvre à partir de mesures fournies par une pluralité de capteurs intégrés dans le dispositif transportable d'évaluation (12).

6. Procédé selon la revendication 5, dans lequel l'étape d'acquisition (130) de données physiologiques comprend au moins l'une de sous-étapes suivantes :
- vérification (131) de l'état de fonctionnement des capteurs ;
- synchronisation (132) des capteurs ;
- suivi (133) de la qualité du signal mesuré ;
- alerte (134) en cas de défaillance d'un ou plusieurs capteurs.

7. Procédé selon l'une quelconque des revendications précédentes, dans lequel l'étape d'acquisition (130) de données physiologiques de l'opérateur est mise en œuvre lors d'une activité de l'opérateur autre que l'interaction avec le dispositif transportable d'évaluation, de préférence selon une durée minimale prédéterminée.

8. Procédé selon l'une quelconque des revendications précédentes, dans lequel le niveau de fatigue de l'opérateur est déterminé par un ou plusieurs algorithmes de traitement des données physiologiques et avantageusement des données contextuelles.

9. Procédé selon l'une quelconque des revendications précédentes, dans lequel l'étape de communication (150) du niveau de fatigue comprend l'affichage de ce niveau de fatigue à l'opérateur et/ou le transfert de ce niveau de fatigue à un dispositif externe.

10. Procédé selon l'une quelconque des revendications précédentes, comprenant en outre une étape (160) de transfert sécurisé des données acquises et/ou déterminés à moins l'un des éléments choisi dans le groupe comprenant :
- un dispositif de stockage externe ;
- un dispositif mobile avec capacité de stockage ;
- un serveur central ;
- un autre dispositif transportable d'évaluation (12).

11. Procédé selon l'une quelconque des revendications précédentes, comprenant en outre une étape (170) d'analyse de fonctionnement du système transportable d'évaluation (12) comprenant la vérification d'au moins un élément choisi dans le groupe comprenant :
- espace de stockage disponible ;
- performances de calcul ;
- état d'une batterie interne ;
- connectivité avec un serveur central (14) et/ou avec un autre dispositif transportable d'évaluation (12) ;
- état de fonctionnement des composants matériels ou logiciels mis en œuvre lorsqu'un dysfonctionnement est détecté

12. Procédé selon l'une quelconque des revendications précédentes, comprenant en outre une étape (180) de mise à jour au moins d'une partie du dispositif transportable d'évaluation choisie dans le groupe comprenant :
- des logiciels mis en œuvre par le dispositif transportable d'évaluation (12) ;
- des algorithmes de traitement de données pour déterminer le niveau de fatigue ;
- la nature des données contextuelles.

13. Procédé selon l'une quelconque des revendications précédentes, dans lequel l'opérateur est affecté à une mission et dans lequel le procédé d'évaluation est mis en œuvre avant la mission de l'opérateur.

14. Procédé selon l'une quelconque des revendications précédentes, mis en œuvre lorsque l'opérateur fait un débriefing de sa mission future ou effectue une activité habituelle relative à ses tâches habituelles et distinctes de sa mission.

15. Système d'évaluation (10) du niveau de fatigue d'un opérateur, comprenant des moyens (12, 14) configurés pour mettre en œuvre le procédé selon l'une quelconque des revendications précédentes.
